Europäisches Patentamt

**European Patent Office**

**Office européen des brevets**

(11) Publication number: **0 149 431**
**A2**

# (12) EUROPEAN PATENT APPLICATION

(21) Application number: **84830285.7**

(22) Date of filing: **23.10.84**

(51) Int. Cl.⁴: **A 61 N 1/04**
**A 61 N 1/362**

(30) Priority: **25.10.83 IT 1189083**

(43) Date of publication of application:
**24.07.85 Bulletin 85/30**

(84) Designated Contracting States:
**AT BE CH DE FR GB LI LU NL SE**

(71) Applicant: **C.B. BIOELETTRONICA S.r.l.**
**Viale Galileo Ferraris, 111**
**I-50019 Sesto Fiorentino Firenze(IT)**

(72) Inventor: **Grassi, Gino**
**Via Imbriani 21**
**I-50019 Sesto Fiorentino Firenze(IT)**

(72) Inventor: **Borghi, Enzo**
**Via Romagnoli 15**
**I-40054 Budrio Bologna(IT)**

(74) Representative: **Lanzoni, Luciano**
**c/o BUGNION S.p.A. Via Farini, 37**
**I-40124 Bologna(IT)**

(54) **Active anchored intracavitary electrocatheter with retractible spring wire.**

(57) The patent relates to an electrocatheter for cardiac stimulation of the retractible active fixation type, the main characteristics of which are: the path followed at the time of anchoring causes no deformation; extraction of the wire for active anchoring to the cardiac muscle is easy; blood and organic liquids are prevented from penetrating into the stimulating head.

The construction throughout with metal components ensures easy use of the catheter without alterations in the functional ability thereof, for a large number of anchoring and withdrawal operations.

EP 0 149 431 A2

FIG1

FIG4

## Active anchored intracavitary electrocatheter with retractible spring wire

Electrocatheters of many types have been proposed for permanent cardiac stimulation and, on each occasion, attempts have been made to solve the various electrophysiological problems that arise as experience in this field increases.

One of the problems that accounts for a good ten per cent of permanent cardiac stimulator post-installation complications has been the change in position of the electrocatheter which, at the offset, was only rested between the carneous trabeculae or, at any rate, against the wall of the chamber to be stimulated.

Various solutions have been adopted, the most certain of which has been seen to be the realization of active fixation electrocatheters whose head is so constructed as to hook on to or be inserted in the muscle to be stimulated.

A variant to this mode of anchoring is the distal electrode constructed with a spring wire made of platinum-iridium or similar metals which, at the time of insertion into the vein, retracts into the body of the head.  Once the electrode is in position, the anchoring wire is thrust outside and, at the same time, screwed to the muscle.

With this system, the electrocatheter stays fixed to the wall both mechanically and electrically.

Though many solutions have been proposed for this type of electrode, certain defects have not yet been eliminated.

For example, the anchoring wire is inclined to spring upwards since it is guided by the spiral of the conductor that tends to load and, therefore, once the force required for the rotation and the extraction of the anchoring turns is reached, to unload suddenly.

Another fault is unsatisfactory sealing of the spring wire against the infiltration of blood into the body of the head in which the movement thereof takes place. Screwing and unscrewing cannot be effected more than a very few times.

Proposed herein is, therefore, a version of the said type of electrocatheter that is free from the faults that are to be found with the majority of catheters currently on the market.

The general principle of the invention is illustrated on the accompanying tables of drawings, in which :

- Figure 1 shows, in a longitudinal sectional view, the head of the electrocatheter in question;
- Figures 2 and 3 show internal constructional details of the said head;
- Figure 4 shows, in a general view, the connector;
- Figures 5 and 6 show constructional details of the element depicted in Figure 4.

The head and the connector are made in such a way that the rotating element and the anchoring wire are mechanically and electrically connected one to the other by means of a spiral that acts as the conductor.

The group constituted by the rotating element, the spiral conductor and the anchoring wire is free to rotate inside an external spiral that links the body of the head to the body of the connector.

The said external spiral serves as a guide that, though flexible, is not easily deformed in a radial direction, inside which the spiral conductor is able to slide and rotate with ease regardless of whatever the form thereof may, for installation reasons, be.

Prevention against the infiltration of blood is ensured by a silicone rubber sealing ring that permits the rotation and axial sliding motion of the mechanism that is thrust outside.

Detailed consideration will now be given to the mechanism to which brief reference has been made above.

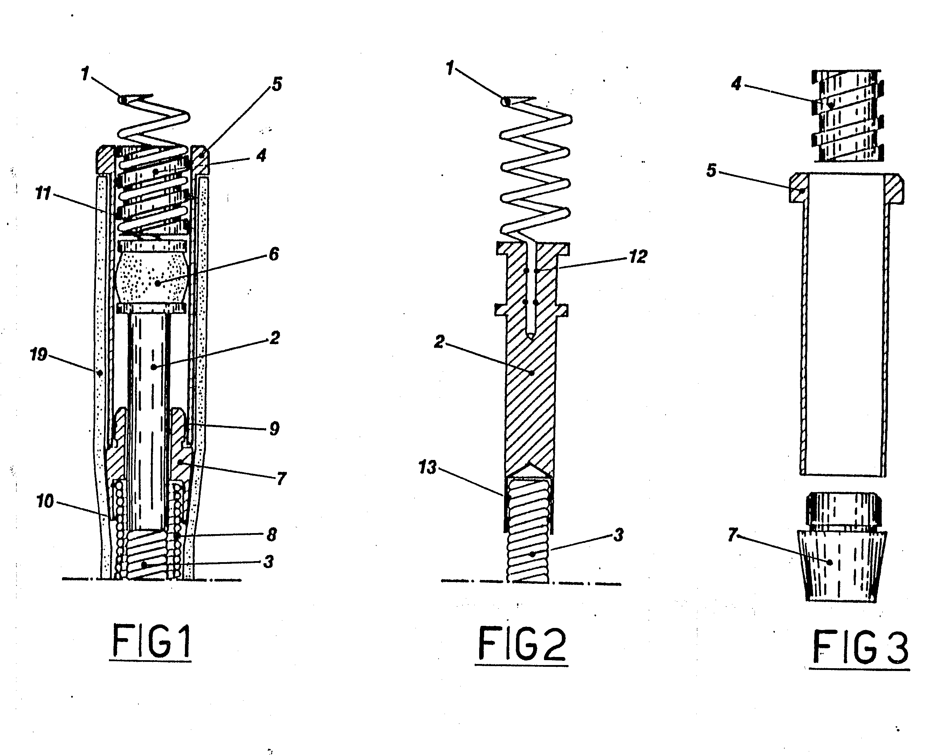

With reference to Figure 1, the mechanism of the stimulating head of the electrocatheter can be seen; in Figure 2, the cylinder 2 connecting the spiral conductor 3 and the anchoring wire 1 is depicted; shown in Figure 3 are the body 5 that contains the retractible head and the endless screw 4 that guides the wire 1 at the time this is being thrust outside, and the lower cylindrical ring 7 that guides the cylinder 2 axially.

The connection cylinder 2 is crimped or welded at 13 to the spiral conductor 3, and at 12 to the anchoring wire 1.

The cylindrical ring 7 is crimped or welded at 9 to the

0149431

body 5 to which is also crimped or welded at 11, the endless screw 4.

When the spiral is rotated counterclockwise, the anchoring wire is guided by the endless screw 4 inside the said body.

The displacement limit is fixed by the abutment of the groove containing the sealing ring 6 against the distal part of the cylindrical guide ring 7.

The length of the displacement is set in such a way that the anchoring wire 1 issues from the head with 1.5 : 2.5 turns, depending on the choice made at the time of construction.

Fashioned proximally in the cylindrical guide ring 7 is a housing 10 in which the external containment spiral 8 is crimped or welded.

The sealing ring 6, despite leaving freedom of movement for axial sliding and rotation, prevents organic liquids from penetrating inside the electrocatheter.

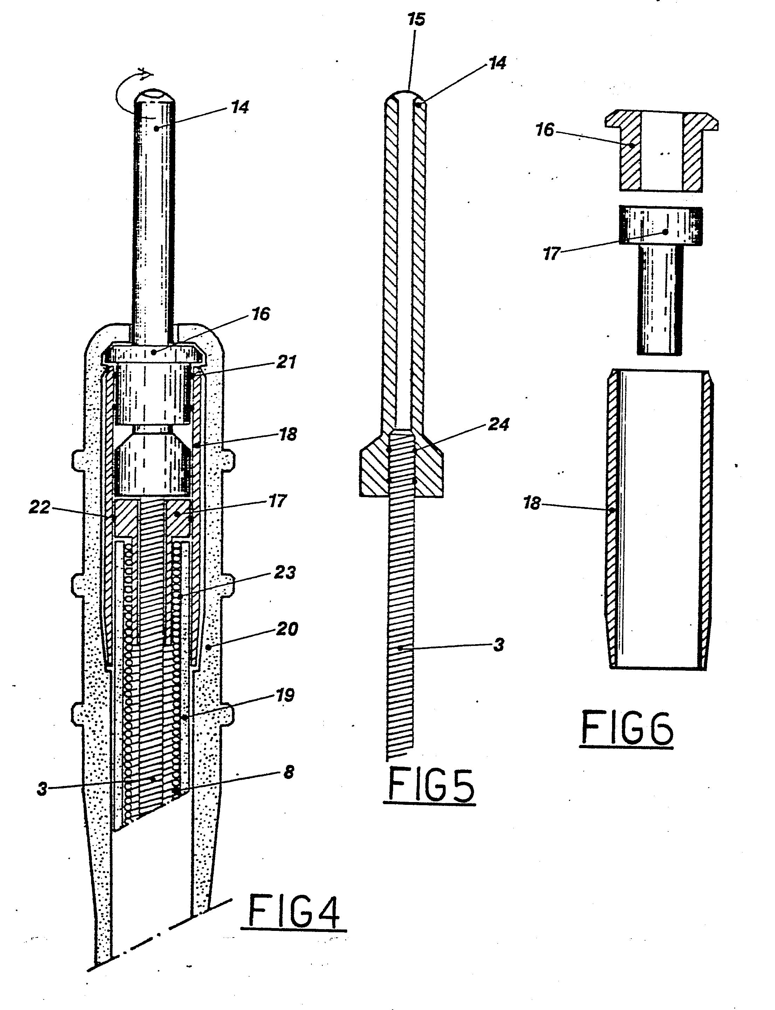

With reference now to Figure 4, a general view is shown of the connector and the rotating element; in Figure 5, the connection of the rotating element to the spiral conductor 3 is illustrated in detail; shown in detail in Figure 6 is the mechanism rotation body 18 together with the lower sealing cylinder 17 inside which rotates the spiral conductor and the upper sealing ring 16 inside which the element 14 rotates.

The said rotating element 14 is connected at 24 (Figure 5) to the conductor spiral by means of crimping or welding in such a way that the group constituted by the rotating element 14, the conductor spiral 3, the body 2 (Figure2) and the anchoring wire 1 can be made to rotate through the rotation of the element 14 in the mechanism containment body 18.

There will be naturally a difference in timing between the rotation of the element 14 and that of the anchoring wire due to the elasticity of the conductor spiral 3 and to the resistance encountered in the tissue as well as due to the curvatures that occur in the electrocatheterization.

The rotation of the element 14 is supported by the bush 16 guided by the body 18 that serves as the container of the complete mechanism.  Contemporaneously, the bush 17 guides in the inside thereof, the conductor spiral 3 and supports on the outside, the external spiral 8.

The bushes 16 and 17 are crimped or welded at 21 and 22, respectively, to the containment body 18 in such a way that the element 14, despite being restrained in position (axially) by the two said bushes, is able to rotate freely.

At 23, the insulating sheath 19, made of biocompatible material, is placed in contact with the external spiral 8 over the full length of the electrocatheter, up as far as covering the body of the stimulating head (Figure 1).

The said sheath placed above the external spiral 8 can be made of biocompatible elastic insulating materials, such as for example, silicone rubber, polyethylene, polyurethane etcetera.

Above the body of the rotating element is moulded or inserted the connector 20 made of silicone rubber or of some other biocompatible elastic material. The said connector will be of the type required for the implantable stimulator to which the electrocatheter has to be connected.

Provided along the axis of the rotating element 14 is a through hole 15 that links with the core of the spiral conductor 3.

This allows the insertion of means constituted by a straightened steel wire, in such a way as to render the electrocatheter rigid during the entering of a vein and, therefore, with the stimulating head in one of the cardiac chambers.

The insertion of the catheter is effected with the head retracted so as not to create hindrances whilst going through the vein.

Once the head is in the desired chamber, it is positioned in a way whereby, stimulating with variable parameter stimulators, it be possible to locate the minor stimulation threshold, contemporaneously with the detection of a satisfactory intracavitary signal.

Positioning can be rendered easier by preforming the stiffening wire inserted in such a way as to cause the head to arrive in the required position through action taken on the proximal part of the electrocatheter to cause the rotation and axial movement thereof.

When the position has been located, the connector of the electrocatheter is kept still and the element 14 (and with this the spiral conductor 3 and the anchoring wire 1) is rotated until the anchoring 1 has been extracted. This normally occurs after six to eight turns of the element 14.

The greater number of turns in comparison with the two exposed turns of the anchoring wire 1 serve to overcome the elasticity of the spiral conductor 3. In order to facilitate the rotation of the element 14, provision can be made for a lever that is fixed radially to this so that the rotation thereof with even one finger be accomplished with ease.

The anchoring wire 1 is constituted by a metal wire having the following characteristics : biocompatibility - a high degree of elasticity and hardness - permanent inability to deform in the case of anchoring manipulations.

The diameter of the wire is generally between 0.15 and 0.33 mm.

Claims:

1. Active anchored electrocatheter with retractible anchoring mechanism, characterized by the fact that the head, made completely of biocompatible metal materials (Pt-Ir or the like), is provided with anchoring mechanism constituted by a natural or activated surface biocompatible material anchoring wire 1 that is controlled and guided by two coaxial spirals 3 and 8.

2. Electrocatheter according to Claim 1, characterized by the fact that of the two mechanism control spirals, the external spiral 8 has a guiding and conduction stimulus function, while the internal spiral 3 has a dual anchoring mechanism extraction and stimulus conduction function.

3. Electrocatheter according to Claim 1, characterized by the fact that the mechanism of the extractible anchoring wire ensures, through one or more biocompatible elastic sealing rings 6, prevention against the infiltration of organic liquids.

4. Electrocatheter according to Claim 1, characterized by the fact that the extraction of the anchoring wire 1 is guided by an endless screw 4 incorporated in the cylindrical body of the head.

5. Electrocatheter according to Claim 2, characterized by the fact that even prior to the extraction of the anchoring wire 1, the electrode is able to stimulate with good characteristics because of the conformation of the cylinder 5, the collar of which can stimulate like a normal passive electrode.

6. Electrocatheter according to Claims 4 and 5, characterized by the fact that the metal component parts (1, 4 and 5) in contact with the blood can be chemically treated in order to obtain both electrochemical activation and a higher degree of biocompatibility (non-thrombogenic membrane covering).

7. Electrocatheter according to Claim 1, characterized by the fact that the form of construction comprising the two spirals, namely one external and one internal, enables there to be a redundance in the stimulus conduction systems whereby maximum reliability for this be ensured.

FIG1

FIG2

FIG3

1/2

0149431

0149431

FIG4

FIG5

FIG6